# EUROPEAN PATENT APPLICATION

(11) **EP 1 580 492 A2**
(43) Date of publication of application: **28.09.2005**
(21) Application number: 05251665.5
(22) Date of filing: 18.03.2005
(51) Int. Cl.: F24F 3/16

(54) **Fluid Treatment System**

(30) Priority: 25.03.2004 GB 0406727
(71) Applicant: Snowball, Malcolm Robert, Essex CM16 6TW (GB)
(72) Inventor: Snowball, Malcolm Robert, Essex CM16 6TW (GB)
(74) Representative: Cawdell, Karen Teresa

(57) **Abstract**

An apparatus for disinfecting air flowing along a duct (10) in an air conditioning or ventilating system, comprises a head portion (12) which is mounted to the outside of the duct, and a body portion (13) extending from the head portion (12) and inserted into the duct through an aperture in its side wall: the body portion supports one or more ultra-violet lamps dispose in respective UV-transparent sleeves (17) and means are provided for causing a flow of e.g. air through the sleeves in order to control the temperature of the lamps.

## Description

The present invention relates to an apparatus for treating fluids and more particularly but not solely to an apparatus for disinfecting air.

It is well known that high intensity UV light has germicidal properties that can be used to disinfect water. EP0202891 discloses a device which utilises these properties. A similar UV technique can be used to disinfect gases and in particular air.

Over the last few years air quality has become of interest to various authorities with regard to the spread of diseases in buildings, particularly in heating and ventilating systems where some or all of the air is re-circulated.

Food producers are also aware that they could potentially be spreading spoilage microorganisms in their food packing areas by re-circulating or ventilating with untreated air.

The issue of air disinfection is becoming important to organisations across a whole host of industries. The technology that has most promise uses UV radiation, provided by UV lamps which are placed in the air duct and kill microorganisms by irradiating them with germicidal radiation in the UVC range.

Known air disinfection systems have suffered from one or more problems, namely:
1. The air circulating in the system is either very cold or quite hot, which severely cuts down the output of the lamps by affecting their internal gas pressure.
2. Installation of a disinfection system is difficult and usually entails the removal of one or more sections of air ducts and replacement with another custom designed section of duct containing UV lamps. The air re-circulating system needs to be shut down during the installation causing inconvenience.
3. The UV lamps quickly become coated with particulates and debris, which are carried in the airflow. This effectively cuts out the germicidal radiation, rendering the disinfection unsatisfactory.
4. Known systems are generally custom built to provide disinfection efficiency based on the airflow. Such systems cannot be easily expanded to cope with increased airflow.
5. Known systems have elongate lamps positioned axially of the airflow, which makes the lamps very difficult to maintain or change.

I have now devised an apparatus for treating fluids which alleviates at least the first of the above-mentioned problems.

In accordance with this invention there is provided an apparatus for treating fluids, the apparatus comprising a head portion and a body portion extending from said head portion, said body portion comprising an elongate sleeve which is transparent to ultra-violet light and is connected at one end thereof to said head portion, an elongate ultra-violet lamp extending axially along the interior of said sleeve, and means for causing a flow of fluid along said sleeve.

The flow of fluid along the sleeve maintains the temperature of the lamp and thus the first of the above-mentioned problems is avoided.

The apparatus can quickly and easily be installed in an existing ventilation duct by forming an aperture in the side wall thereof and inserting the body of the apparatus through the aperture. In this manner, the second of the above-mentioned problems is avoided.

The system can easily be expanded to cope with increased airflow by mounting further apparatus to the duct, thereby alleviating the fourth of the above-mentioned problems.

Preferably the lamp is accessible through the head for maintenance (including removal and replacement)and thus the fifth of the above-mentioned problems is avoided.

Preferably a plurality of sleeves extend substantially parallel to each other from the head, a UV lamp being mounted inside each sleeve.

Preferably the or each sleeve is formed of quartz is are preferably open at both ends.

Preferably a frame extends from the body, the frame supporting the or each sleeve adjacent the distal end(s) thereof.

Preferably in order to overcome the third of the above-mentioned problems, means are provided to clean the or each sleeve.

Preferably the cleaning means comprises a head mounted to the or each sleeve, the or each head being movable axially along the sleeve to clean the latter.

Preferably the or each cleaning head is supported by the frame.

Preferably the or each cleaning head is driven axially by drive means mounted within the head portion of the apparatus.

Preferably the or each cleaning head is attached to the drive means in a manner which allows independent movement of the cleaning head, in order to account for slight misalignment of the head with the sleeve.

Preferably each cleaning head is attached to a support which is driven axially of the sleeves, so that all quartz sleeves are cleaned at the same time.

Preferably, the or each cleaning head comprises a cleaning material of the silicon rubber or fluorocarbon family of materials and preferably has a foam cellular construction.

Preferably the temperature of the or each lamp is controlled by blowing heated or cooled air through the annular space between the sleeve and the surface of the lamp.

The air disinfection system described can be fitted through the side, top or bottom of a rectangular-section duct via an aperture cut into the duct wall without causing a critical pressure drop problem to the air conditioning system. The same unit can be fitted in a similar manner into a circular-section duct. The disinfection system can be mounted with the lamps parallel and perpendicular to the airflow or mounted with the lamps in series but perpendicular to the airflow.

Also in accordance with this invention, there is provided a duct for an air conditioning or ventilation system, the duct comprising a tubular side wall defining an airflow duct, an aperture being formed in the side wall of the duct, the body portion of an apparatus as hereinbefore defined extending through the aperture into the airflow duct, the head portion of the apparatus being mounted externally of said side wall.

Preferably said body of the apparatus comprises means for causing a flow of gas along said sleeve.

Preferably a filter is mounted in the gas flow, upstream of the sleeve, in order to prevent the internal wall of the sleeve and the exterior of the lamp from being coated with dust.

Preferably the gas flowing along the sleeve vents into the airflow duct through an open distal end of the sleeve.

Preferably the gas flowing along the sleeve is air, preferably drawn into the head portion of the apparatus from the atmosphere.

Also in accordance in accordance with this invention, there is provided a method of fitting a disinfection apparatus to a duct of an air conditioning or ventilation system, the method comprising:
providing an apparatus comprising a head portion and an body portion extending from the head portion, said body portion comprising an elongate ultra-violet lamp extending axially away from the head portion;
forming an aperture in a sidewall of the duct;
inserting the body portion of the apparatus through the aperture; and
mounting the head portion of the apparatus external to said duct.

Preferably the apparatus comprises a plurality of lamps mounted in parallel in a common plane, the apparatus being mounted to the duct such that said plane extends substantially normal to the airflow along the duct.

Embodiments of this invention will now be described by way of examples only and with reference to the accompanying drawings, in which:
Figure 1 is an isometric view of an air disinfection apparatus in accordance with this invention, when mounted to a rectilinear ventilation duct;
Figure 2 is a perspective view of the assembly of Figure 1;
Figure 3 is a longitudinal sectional view through a cleaning head of the apparatus of Figures 1; and
Figure 4 is an isometric view of an air disinfection apparatus in accordance with this invention, when mounted to a curvilinear ventilation duct.

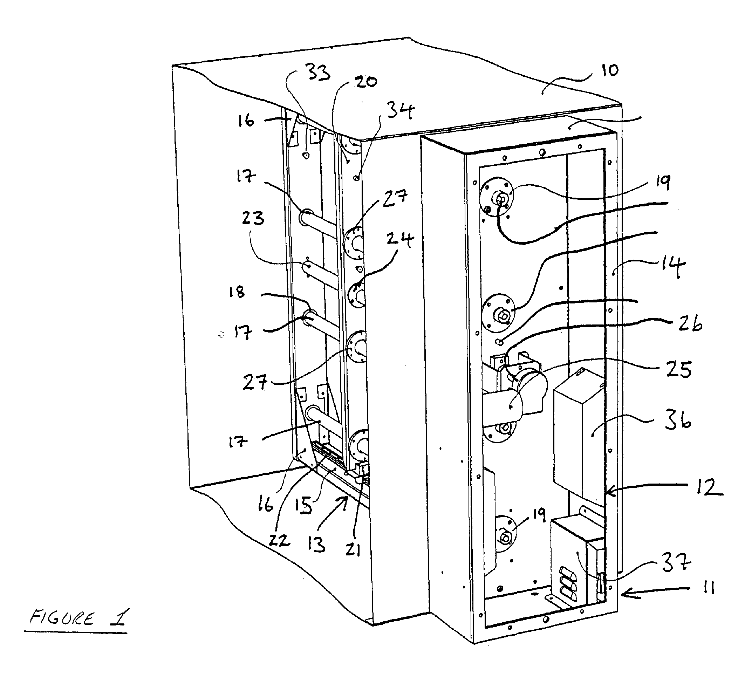

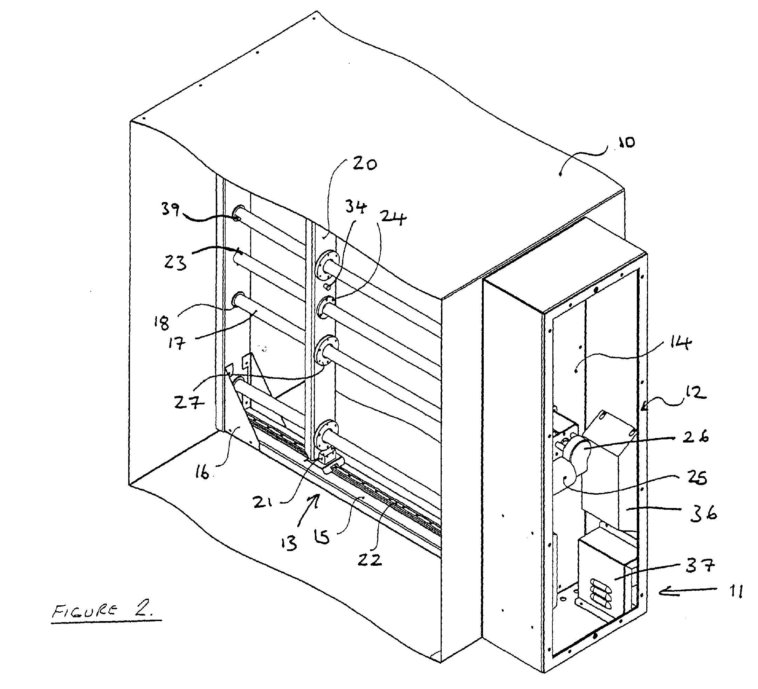

Referring to Figures 1 and 2 of the drawings, there is shown a square or rectangular walled tubular duct 10 of a conventional ventilation system, fitted with an air disinfection apparatus 11 which comprises a head portion 12 and a body portion 13 extending from the head portion 12. The apparatus 11 is mounted to the duct 10, such that the body portion 13 thereof extends through an aperture formed in a sidewall of the duct 10.

The head portion 12 of the apparatus 11 comprises a rectangular housing 14 attached to the exterior of the wall of the duct 10. The body portion 13 of the apparatus 11 comprises a square or rectangular frame 15 attached along its inner side to the rear wall of the housing 14 of the head 12. The frame is strengthened by corner brackets 16 to form a rigid structure. Apertures formed in the rear wall of the housing 14 of the head 12 are aligned with corresponding apertures formed in the outer wall of the frame 15. An elongate open ended quartz sleeve 17 extends through each aperture in the head 12 and through the corresponding aperture formed in the outer wall of the frame 15.

The sleeves 17 are supported at their distal ends by PTFE collars 18 fitted in respective apertures in the outer side edge of the frame 15. The proximal ends of the sleeves 17 are clamped to the head 12 by collars 19 fitted in respective apertures in the rear wall of the housing 14 of the head 12.

An elongate bar 20 is slidingly mounted at its opposite ends to the upper and lower side edges, respectively, of the frame 15. The bar 20 extends perpendicular to the longitudinal axis of the sleeves 17, which extend through respective apertures formed in the bar 20. The bar 20 comprises bearings 21 at its opposite ends, which are attached to runners 22 extending along the upper and lower side edges of the frame 15.

An elongate, externally screw-threaded shaft 23 extends between the head 12 and the outer side edge of the frame 15, parallel to the sleeves 17. The externally screw-threaded shaft 23 extends through an internally screw-threaded collar 24 in the bar 20. The proximal end of the externally screw-threaded shaft 23 is connected inside the housing 14 to a motor 25 by a gearbox 26. In this manner, the bar 21 can be driven back and forth, maintaining a substantially perpendicular position with respect to the sleeves 17.

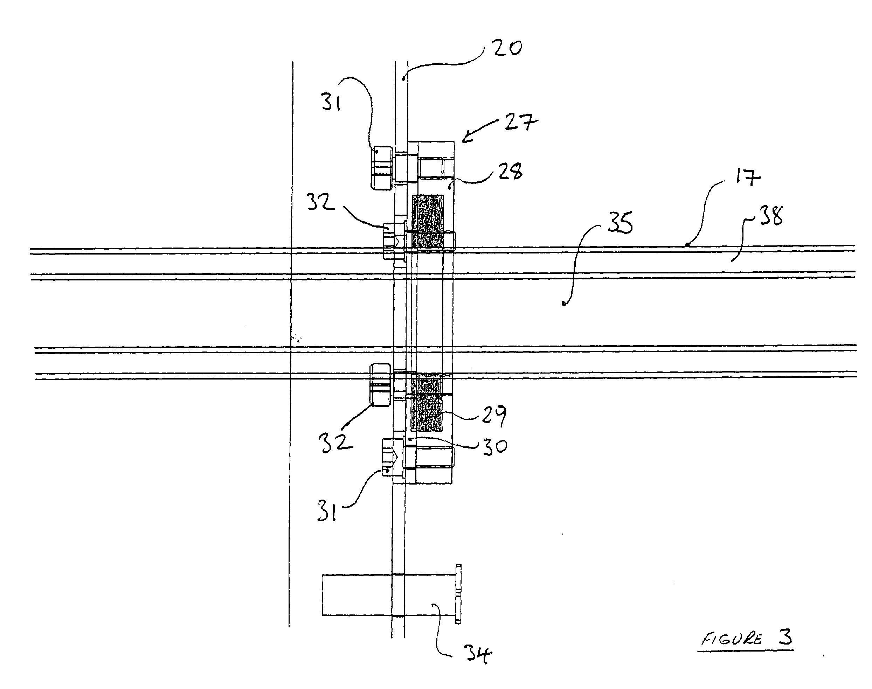

Referring to Figure 3 of the drawings, the bar 20 carries a plurality of cleaning heads 27 surrounding respective quartz sleeves 17. The heads 27 each comprise a body 28 and an annular cleaning ring 29, which is clamped and secured by a collar 30 and bolts 31. The cleaning head 27 is attached to the bar 20 by shoulder bolts 32 which extend through over-sized apertures in the bar 20, so that the cleaning head 27 can adjust its position relative to the quartz sleeve 17. The cleaning heads 27 are designed to have clearance over the quartz tube such that only the cleaning ring 29 engages the quartz sleeves 17.

The material of the cleaning rings 29 is pliable yet resilient, and is formed of a material that is resistant to UVC radiation and is wide enough to make a broad contact with the quartz sleeves 17.

The cleaning rings 29 have a central orifice of a diameter which is slightly smaller than the diameter of the quartz sleeves 17, such that when the cleaning rings 29 are positioned over the quartz sleeves 17 they lightly grip the quartz sleeves 17. When the cleaning rings 29 are driven along the surface of the quartz sleeve 17 by rotation of the motor 25, they produce a wiping action and hence clean the quartz sleeves 17. A foam cellular construction of the cleaning rings 29 produce a scrubbing action as they move across the surface of the quartz sleeves 17 as the abrasive edges of the cells engage on the quartz sleeves 17. The rotation of the motor 25 is interrupted and reversed by limit switches 33 on the inner and outer side edges of the frame 15. The switches 33 are engaged by axially adjustable projections 34 carried on the bar 20.

The generation of UVC germicidal radiation is provided by UV lamps 35, which are placed inside respective quartz sleeves 17 and which are driven by a lamp ballast 36. The lamps 35 are of the low-pressure type, which runs at a low skin temperature and thus debris in the air duct will not fuse onto the surface of the quartz sleeves 17. The ballast 36 is of the high frequency electronic type for high efficiency and low running costs.

The apparatus comprises a lamp conditioning system to maintain the optimum operating temperature of the lamps 35. This apparatus comprises a fan unit 37 to ventilate the cavity between the lamps 35 and the quartz sleeves 17 and to also either heat or cool the air entering this cavity to maintain the temperature and keep the lamps at optimum performance, irrespective of the ambient temperature.

The fan unit 37 comprises an impeller which draws air in from the atmosphere into the housing 14 to create a positive pressure inside the housing 14. Means for filtering the air is provided in the unit to prevent the accumulation of dust inside the apparatus. The pressurised air inside the housing 14 flows into the proximal end of the sleeves 17 and enters the cavities 38 surrounding the lamps 35. The temperature of the air is controlled by a temperature detector 39 fixed inside the cavity 38 surrounding one of the lamps 35 at the distal end thereof.

The heating and cooling device preferably comprises a Peltier device, which when orientated correctly and powered will either cool or heat air moving across its surface.

The lamps 35 lie in a common plane which extends normal to the direction of airflow along the duct 10.

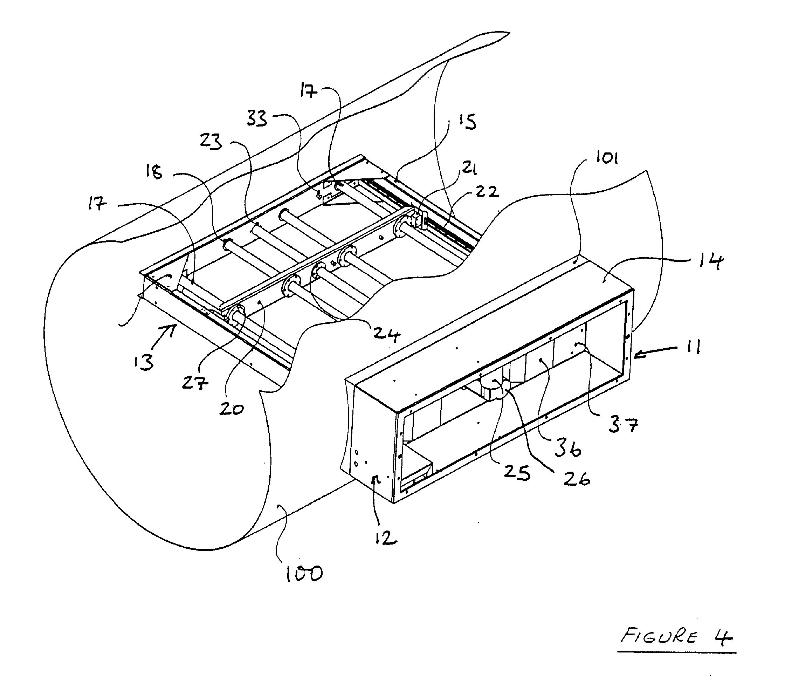

Referring to Figure 4 of the drawings, there is shown an alternative embodiment and like parts are given like reference numerals. In this embodiment, the duct 100 comprises a circular or elliptical wall. The air disinfection apparatus 11 is mounted to the duct 100, such that the body portion 13 thereof extends through an aperture formed in a sidewall of the duct 100. A spacer 102 adapts the flat rear wall of the housing 14 of the head 12 of the apparatus 11 to fit the curved duct 100. In this embodiment, the lamps 35 lie in a common plane which extends parallel to the direction of airflow along the duct 100.

A radiation detector (not shown) in the duct 100 monitors the UVC radiation from the lamps 35 and feeds back information to a control unit on the lamp condition and radiation intensity.

The apparatus described is self-contained and can be fitted through a rectangular aperture cut into a duct wall. The apparatus is not affected by duct air temperature, and can be fitted without shutting the ventilation system down and does not need custom-built ducts. Additionally, it is totally modular and several units can be installed in parallel or series to provide higher disinfection rates. The unit is self-cleaning, easily to maintain and will fit substantially all shapes of ducts.

## Claims

1. An apparatus for treating fluids, the apparatus comprising a head portion and a body portion extending from said head portion, said body portion comprising an elongate sleeve which is transparent to ultra-violet light and is connected at one end thereof to said head portion, an elongate ultra-violet lamp extending axially along the interior of said sleeve, and means for causing a flow of fluid along said sleeve.

2. An apparatus as claimed in claim 1, in which said lamp is accessible through said head portion.

3. An apparatus as claimed in claim 1 or 2, in which said body portion comprises a plurality of said sleeves extending generally parallel to each other from said head portion, a said ultra-violet lamp being disposed within each said sleeve.

4. An apparatus as claimed in any preceding claim, in which the or each said sleeve is formed of quartz.

5. An apparatus as claimed in any preceding claim, in which the or each said sleeve is open at both ends thereof.

6. An apparatus as claimed in any preceding claim, in which said body portion comprises a frame which supports the or each said sleeve adjacent the ends thereof.

7. An apparatus as claimed in any preceding claim, comprising means for cleaning the or each said sleeve.

8. An apparatus as claimed in claim 7, in which said cleaning means comprises a cleaning head mounted to the or each sleeve, the or each said cleaning head being moveable axially along its sleeve to clean said sleeve.

9. An apparatus as claimed in claim 7 appended to claim 6, in which the or each cleaning head is supported by said frame.

10. An apparatus as claimed in claim 8 or 9, comprising drive means mounted within said head portion and arranged to drive the or each cleaning head axially along its said sleeve.

11. An apparatus as claimed in any one of claims 8 to 10, in which each cleaning head is attached to a support which is driven axially of said sleeves for cleaning all said sleeves at the same time.

12. An apparatus as claimed in any preceding claim, comprising means for controlling the temperature of the or each said lamp by blowing heated or cooled air through the respective sleeve in the space between the inner surface of the sleeve and the outer surface of the lamp.

13. A duct for an air conditioning or ventilating system, the duct comprising a tubular side wall defining an airflow duct, an aperture being formed in the side wall of the duct, and an apparatus as claimed in any preceding claim having its said body portion extending through said aperture and into said airflow duct, and its said head portion mounted externally of said wall.

14. A duct as claimed in claim 13, in which said body portion of said apparatus comprises means for causing a flow of air or gas along the or each said sleeve.

15. A duct as claimed in claim 13, arranged for the air or gas flowing along the or each sleeve to vent into said airflow duct through an open, downstream end of the sleeve.

16. A duct as claimed in claim 14 or 15, in which said flow causing means is arranged to draw in air from the atmosphere and cause it to flow along the or each sleeve.

17. A duct as claimed in any one of claims 13 to 16, in which said apparatus comprises a plurality of said lamps mounted in parallel in a common plane, the apparatus being mounted to said duct such that the airflow passes along the duct in a direction normal to said plane.

18. A method of fitting a disinfection apparatus to a duct of an air conditioning or ventilation system, the method comprising:
providing an apparatus comprising a head portion and an body portion extending from the head portion, said body portion comprising an elongate ultra-violet lamp extending axially away from the head portion;
forming an aperture in a sidewall of the duct;
inserting the body portion of the apparatus through the aperture; and
mounting the head portion of the apparatus external to said duct.
